# EUROPÄISCHE PATENTANMELDUNG

(11) **EP 2 732 812 A1**
(43) Veröffentlichungstag der Anmeldung: **21.05.2014**
(21) Anmeldenummer: 13192621.4
(22) Anmeldetag: 13.11.2013
(51) Int. Cl.: A61K 9/20, A61K 47/32, A61K 47/38, A61K 31/425

(54) **Pramipexol-Retardtablettenformulierung**

(30) Priorität: 15.11.2012 DE 102012110981
(71) Anmelder: Aristo Pharma GmbH, 13435 Berlin (DE)
(72) Erfinder: Langer, Dr. Britta, 10965 Berlin (DE); Walz, Stephan, 14129 Berlin (DE)
(74) Vertreter: Gille Hrabal

(57) **Zusammenfassung**

Die vorliegende Erfindung betrifft eine Retardtablettenformulierung, umfassend 0,05 Gew.-% bis 1 Gew.-% des Wirkstoffs Pramipexol oder eines pharmazeutisch verträglichen Salzes davon, eine Matrix, umfassend 15 bis 35 Gew.-% Hydroxypropylmethylcellulose, 20 bis 48 Gew.-% Polyvinylacetat und 4,75 bis 11,40 Gew.-% Polyvinylpyrrolidon sowie (c) mindestens einen bei der Tablettenformulierung üblichen Bestandteil. Ebenso betrifft die vorliegende Erfindung eine Matrixtablette umfassend die Retardtablettenformulierung, sowie ein Verfahren zur ihrer Herstellung.

## Beschreibung

Die vorliegende Erfindung betrifft eine Retardtablettenformulierung, umfassend 0,05 Gew.-% bis 1 Gew.-% des Wirkstoffs Pramipexol oder eines pharmazeutisch verträglichen Salzes davon, eine Matrix, umfassend 15 bis 35 Gew.-% Hydroxypropylmethylcellulose, 20 bis 48 Gew.-% Polyvinylacetat und 4,75 bis 11,40 Gew.-% Polyvinylpyrrolidon sowie (c) mindestens einen bei Tablettenformulierungen üblichen Bestandteil. Ebenso betrifft die vorliegende Erfindung eine Matrixtablette umfassend die Retardtablettenformulierung, sowie ein Verfahren zur ihrer Herstellung.

### Beschreibung

Der Wirkstoff Pramipexol ((S)-2-Amino-6-(propylamino-4,5,6,7-tetrahydrobenzothiazol; Pramipexol Base) der folgenden Formel (I) 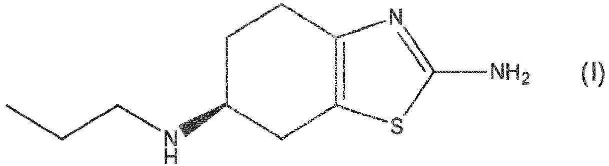 ist ein Dopaminagonist mit hoher Spezifität an D_{2/3}-Dopaminrezeptoren. Er ist unter anderem bekannt aus der EP 0 186 087 A1. Pramipexol wird am häufigsten in der Form des Dihydrochloridmonohydrates insbesondere zur Behandlung der Parkinson-Erkrankung und des Restless-Legs-Syndroms verwendet.

Wie auch bei anderen bekannten Parkinsonmitteln kann es bei der Behandlung mit Pramipexol zu unerwünschten Nebenwirkungen wie zum Beispiel orthostatische Hypotonie kommen. Viele dieser Nebenwirkungen sind dosisabhängig. Es ist zwar bekannt, dass durch die Veränderung des Wirstofffreisetzungsprofils beispielsweise durch kontrollierte, verlängerte oder verzögerte Freisetzung eine vereinfachte Verabreichung für den Patienten und damit eine Reduzierung der Nebenwirkungen durch Vermeidung von Spitzen der Blutplasmakonzentration resultieren kann. Jedoch ist eine Kontrolle, Verlängerung oder Verzögerung der Freisetzung peroraler Darreichungsformen gerade bei Wirkstoffen, welche eine hohe Löslichkeit in der Magensäure aufweisen, problematisch und kann nur durch eine angepasste Galenik gewährleistet werden. Auch Pramipexoldihydrochlorid Monohydrat weist eine Löslichkeit In Wasser von mehr als 20 mg/mL und In Puffer bei einem pH-Wert von 2,0 bis 7,4 von über 10 mg/mL auf.

Eine Möglichkeit, die Freisetzung gut löslicher Wirkstoffe zu beeinflussen, stellt die Einbettung des Wirkstoffes in eine Matrix dar (auch als Gerüst bezeichnet). Die Freisetzung eines Wirkstoffes aus solch einer oralen Matrixtablette kann je nach Wahl der Matrix über drei unterschiedliche Mechanismen verlaufen.
(i) Werden in der Magensäure lösliche Gerüstbildner verwendet, so geht der in diesem Gerüstbildner homogen dispergierte Wirkstoff gleichzeitig mit der graduellen Auflösung des Gerüstbildners in Lösung und wird freigesetzt.
(ii) Eine andere Methode stellt die Verwendung von hydrophilen Quellstoffen als Gerüstbildner dar. Diese sind in der Lage, in der Flüssigkeit des Magen-Darm-Traktes zu quellen und dadurch ein Gel zu bilden, welches das weitere Eindringen der Flüssigkeit in das Tabletteninnere verlangsamt. Der in einer solchen Tablette enthaltene Wirkstoff muss zunächst von der Flüssigkeit gelöst werden, dann durch die Gelschlcht hindurch diffundieren, um anschließend freigesetzt zu werden. Die Freisetzungsgeschwindigkeit wird somit durch den Eintritt der Flüssigkeit in die Tablette und die Diffusion des Wirkstoffs durch die gebildete Gelschicht bestimmt. Gleichzeitig wird diese Gelschicht jedoch auch durch Erosion abgebaut, so dass hier die Freisetzung teilweise auch durch den oben beschriebenen Mechanismus (i), der Auflösung des Gerüstbildners, beschrieben werden kann.
(iii) Wird ein in der Flüssigkeit des Magen-Darm-Traktes unlöslicher Gerüstbildner verwendet, erhält man eine porenartige Matrix. Die Verdauungssäfte lösen zunächst den an der Oberfläche der Tablette lokalisierten Wirkstoff und dringen dann in das Innere der Tablette vor. Der Wirkstoff wird durch Diffusion nach dem 1. Fick'schen Gesetz freigesetzt. Die Freisetzung hängt vom Masseverhältnis zwischen Arzneistoff und Gerüstsubstanz, von der Arzneistoffkonzentration und von der Anzahl und Struktur der Kapillaren und der Hohlräume im Gerüst ab (R. Voigt, Pharmazeutische Technologie; 9. Auflage, Deutscher Apotheker Verlag Stuttgart 2000, S. 260). Dies hat zur Folge, dass bei vielen Retardtabletten die Menge an Gerüstsubstanz der Wirkstoffmenge angepasst werden muss.

Es sind bereits einige Ansätze zur verlangsamten Freisetzung des Wirkstoffes Pramipexoldihydrochlorid Monohydrat bekannt. So vertreibt Boehringer Ingelheim das Präparat Sifrol^{®} als Retardtablette mit unterschiedlichen Dosierungen. Die Formulierungen enthalten Hydroxypropylmethylcellulose, Maisstärke, Carbomer^{®} 941, hochdisperses Siliciumdioxid und Magnesiumstearat. Die unterschiedlichen Dosierungen des Wirkstoffes werden in unterschiedlichen Tablettengewichten vertrieben, um ein entsprechend gleichbleibendes Freisetzungsprofil zu gewährleisten. Die folgende Tabelle 1 gibt die Tablettengewichte und die entsprechenden Wirkstoffmengen der derzeit auf dem Markt erhältlichen Tabletten wieder.

**Tabelle 1:**

| **Wirkstoffmenge Pramipexol Base (mg)** | **Wirkstoffmenge Pramipexoldihydrochlorid Monohydrat (mg)** | **Wirkstoffmenge Pramipexol Base (Gew.-%)** | **Wirkstoffmenge Pramipexoldihydrochlorid Monohydrat (Gew.-%)** | **Tablettengewicht (mg)** |
|---|---|---|---|---|
| 0,26 | 0,375 | 0,10 | 0,15 | 248 |
| 0,52 | 0,75 | 0,16 | 0,23 | 330 |
| 1,05 | 1,50 | 0,30 | 0,43 | 350 |
| 1,57 | 2,25 | 0,39 | 0,56 | 399 |
| 2,10 | 3,00 | 0,49 | 0,71 | 425 |
| 2,62 | 3,75 | 0,58 | 0,83 | 450 |
| 3,15 | 4,50 | 0,63 | 0,91 | 497 |

Besonders nachteilig Ist es, dass bei der Herstellung dieser Tabletten in Abhängigkeit von der Wirkstoffkonzentration jeweils angepasste Verpressungsutensilien (Stempel, Matrizen etc.) sowie angepasste Konfektionierungsutensilien (Blisterwerkzeuge etc.) benötigt werden. Des Weiteren wird die Einnahme großer Tabletten ohne Zerkauen von vielen Patienten abgelehnt auf Grund der Schwierigkeiten, die beim Schlucken größerer Tabletten (insbesondere die Problematik des Verschluckens) entstehen können. Ein Zerkauen von Retardformullerungen ist problematisch. Die Zerkleinerung führt im Regelfall dazu, dass die Diffusionswege des enthaltenen Wirkstoffes verkürzt werden und/oder Diffusionsbarrieren entfernt werden. Als Folge wird der In der Darreichungsform ursprünglich enthaltene Wirkstoff in relativ kurzer Zeit freigesetzt, wodurch sprunghaft für eine relativ kurze Zeit eine vergleichsweise sehr hohe Plasmakonzentration der Substanz erreicht wird. Gleichzeitig weisen solche Darreichungsformen beim Zerkauen auch einen bitteren Geschmack auf, der von den Patienten als unangenehm empfunden wird.

In der WO 2006/015944 A1 wird eine Retardtablettenformulierung, umfassend Pramipexoldihydrochlorid Monohydrat, beschrieben, welche als Matrix mindestens Quellstärke und ein anionisches Polymer umfasst. Auch die WO 2006/015942 A1 beschreibt Retardtabletten des Wirkstoffs Pramipexoldihydrochlorid Monohydrat, wobei die Matrix der Retardtabletten mindestens ein in Wasser quellbares Polymer umfasst, welches verschieden Ist von Quellstärke. Beide Dokumente beschreiben somit Tabletten, welche eine quellbare und teilweise erodierende Polymermatrix umfassen. Einen ähnlichen Freisetzungsmechanismus des Pramipexoldihydrochlorid Monohydrates wird in der WO 2004/010997 A1 beschrieben, deren Tabletten als Matrix ein hydrophiles Polymer und eine Stärke mit einer Zugfestigkeit von mindestens 0,15 kN cm⁻² aufweist. In der WO 2004/010999 A1 und der WO 2004/010998 A1 werden in Verdauungssäften unlösliche Überzüge von Tabletten, deren Kerne aus hydrophilen Matrices bestehen, beschrieben. Die WO 2011/037976 A2 beschreibt zwar allgemein, dass hydrophobe Polymere in der Tablettenmatrix mit hydrophilen und/oder quellbaren Polymeren vermischt werden können. Jedoch gibt dieses Dokument keinen Hinweis darauf, welche konkreten Auswirkungen diese Beimischung auf das Freisetzungsprofil des Pramipexols in Tabletten unterschiedlicher Wirkstoffkonzentrationen hat. Ebenso wird in der EP 1 138 320 A2 eine Matrix beschrieben, welche ein in den Verdauungssäften lösliches, ein quellbares und ein unlösliches Polymer umfasst, jedoch werden auch hier keine Auswirkungen auf Freisetzungsprofile bei unterschiedlichen Wirkstoffkonzentrationen beschrieben.

Ausgehend von diesem Stand der Technik lag der vorliegenden Erfindung somit insbesondere die Aufgabe zu Grunde, eine Retardtablettenformulierung, umfassend Pramipexol oder ein pharmazeutisch verträgliches Salz davon bereit zu stellen, welche mindestens einen der oben genannten Nachteile des Standes der Technik behebt. Dabei sollten insbesondere Retardtablettenformulierungen bereitgestellt werden, welche selbst bei variierender Wirkstoffkonzentration in einem Bereich von 0,05 bis 1 Gew.-% des Pramipexols berechnet als freie Base, bezogen auf das Gesamttablettengewicht, im Wesentlichen das gleiche Freisetzungsprofil aufweisen. Es sollen also das Tablettengewicht und damit insbesondere auch die Tablettengröße konstant und daher unabhängig von der genannten Wirkstoffkonzentration sein.

Diese Aufgaben werden gelöst durch die Bereitstellung einer Retardtablettenformulierung, umfassend Pramipexol oder ein pharmazeutisch verträgliches Salz davon, dadurch gekennzeichnet, dass sie (a) 0,05 Gew.-% bis 1 Gew.-% des Pramipexols berechnet als Base, (b) eine Matrix und (c) mindestens einen bei Tablettenformulierungen üblichen Bestandteil umfasst, wobei die Matrix 15 bis 35 Gew.-% Hydroxypropylmethylcellulose, 20 bis 48 Gew.-% Polyvinylacetat und 4,75 bis 11,40 Gew.-% Polyvinylpyrrolidon, jeweils bezogen auf das Gesamtgewicht der Retardtablettenformulierung, umfasst.

Ebenso werden diese Aufgaben gelöst durch die Bereitstellung einer Matrixtablette, welche die erfindungsgemäße Retardtablettenformulierung umfasst, und ein Verfahren zu Ihrer Herstellung.

### Retardtablettenformulierung

Die erfindungsgemäße Retardtablettenformulierung umfasst Pramipexol oder ein pharmazeutisch verträgliches Salz davon und ist dadurch gekennzeichnet, dass sie (a) 0,05 Gew.-% bis 1 Gew.-% des Pramipexols berechnet als Base, (b) eine Matrix und (c) mindestens einen bei Tablettenformulierungen üblichen Bestandteil umfasst.

Alle angegebenen Gew.-% beziehen sich, soweit nicht anders angegeben, auf das Gesamtgewicht der Tablette.

Unter einer verlangsamten oder retardierten Freisetzung im Sinne der Erfindung wird eine Freisetzung verstanden, bei der der Wirkstoff kontinuierlich über einen bestimmten Zeitraum, Insbesondere über maximal 24 h frei gesetzt wird. Dies kann über den Zeitraum schneller oder langsamer erfolgen. Insbesondere steht der Begriff der verlangsamen oder retardierten Freisetzung im Gegensatz zu einer sofortigen Freisetzung des Wirkstoffes. Eine Retardtablette erlaubt daher Insbesondere eine Reduzierung der Einnahmehäufigkeit einer Tablette und ermöglicht damit eine Vereinfachung für den Patienten, Insbesondere ermöglicht eine Retardtablette, dass ein Patient diese nur einmal pro Tag einnehmen muss. Dies bedeutet eine substantielle Verbesserung der Patientencompliance.

### (a) Wirkstoff Pramipexol

Als Wirkstoff umfasst die erfindungsgemäße Retardtablettenformulierung Pramipexol oder ein pharmazeutisch verträgliches Salz davon. Unter "Pramipexol" wird die Pramipexol Base, welche in der Formel (I) wiedergegeben ist, verstanden. Als "pharmazeutisch verträglich" werden im Sinne der Erfindung Substanzen bezeichnet, welche geeignet sind, in Kontakt mit menschlichem oder tierischem Gewebe zu kommen, ohne dabei insbesondere toxische, reizende oder allergische Reaktionen hervorrufende Wirkungen zu erzeugen.

Pharmazeutisch verträgliche Salze des Pramipexols gehören bevorzugt der BCS-Klasse I (Biopharmaceutics Classlflcatlon System) an (siehe beispielsweise: Guideline on the investigation of bioequivalence (CPMP/EWP/QWP/1401/98 Rev. 1/ Corr; Januar 2010), European Medicines Agency). Insbesondere weist das pharmazeutisch verträgliche Salz des Pramipexols eine hohe Löslichkeit in einem wässrigen Medium und ein gutes Permeationsvermögen auf. Dies bedeutet, dass der Arzneistoff sich in seiner höchsten therapeutischen Einzeldosis in 250 Milliliter Pufferlösung des physiologischen pH-Berelchs von 1 bis 6,8 vollständig auflösen lässt (dabei müssen mind. drei Pufferlösungen getestet werden, vorzugsweise pH 1,2; 4,5 und 6,8) und zu mehr als 85 % resorbiert wird.

Besonders bevorzugt wird das pharmazeutisch verträgliche Salz des Pramipexols ausgewählt aus der Gruppe, bestehend aus Pramipexoldihydrochlorid Monohydrat und Pramipexoldihydrochlorid. In einer besonders bevorzugten Ausführungsform ist das pharmazeutisch verträgliche Salz des Pramipexols Pramipexoldihydrochlorid Monohydrat.

Die erfindungsgemäße Retardtablettenformulierung umfasst 0,05 Gew.-% bis 1 Gew.-%, bevorzugt 0,06 Gew.-% bis 0,95 Gew.-%, ganz besonders bevorzugt 0,07 Gew.-% bis 0,90 Gew.-% des Pramipexols als berechnet Base. Umfasst die erfindungsgemäße Retardtablettenformulierung ein pharmazeutisch verträgliches Salz des Pramipexols, so bedeutet die Formulierung, der erfindungsgemäß angegebene Gewichtsanteil des Pramipexols berechnet als Base betrage 0,05 Gew.-% bis 1 Gew.-%, dass auf das molare Äquivalent der Pramipexol Base statt auf das tatsächlich enthaltene pharmazeutisch verträgliche Salz abgestellt wird. Dies bedeutet beispielsweise, dass die Retardtablettenformulierung bei der Angabe 0,05 Gew.-% bis 1 Gew.-% des Pramipexols berechnet als Base 0,07 bis 1,43 Gew.-% an Pramipexoldihydrochlorid Monohydrat umfasst. Insgesamt ist die erfindungsgemäße angegebene Menge an Pramipexol Base somit so zu verstehen, dass die erfindungsgemäße Retardtablettenformulierung eine gegebenenfalls theoretisch freisetzbare Menge an Pramipexol Base enthält, welche aus dem Verhältnis der molaren Gewichte des pharmazeutisch verträglichen Pramipexol Salzes und der Pramipexol Base berechnet werden kann. Die Formulierung ist nicht so zu verstehen, dass das Pramipexol tatsächlich immer als Base in der erfindungsgemäßen Retardtablettenformulierung vorliegt.

Besonders bevorzugt umfasst die erfindungsgemäße Retardtablettenformulierung 0,07 Gew.-%, 0,15 Gew.-%, 0,30 Gew.-%, 0,45 Gew.-%, 0,60 Gew.%, 0,75 Gew.-% oder 0,90 Gew.-% des Pramipexols berechnet als Base. Dabei ist es besonders bevorzugt, wenn die Tabletten jeweils ein Gesamtgewicht von 350 mg aufweisen.

Die Partikelgröße eines Wirkstoffes kann Einfluss auf die Dosierung einer festen Verabreichungsform haben. So hängt beispielsweise die Einheitlichkeit des Wirkstoffgehaltes In den Matrixtabletten auch von der Partikelgröße des Wirkstoffes und dessen Größenverteilung ab. Dieser Effekt Ist umso mehr zu berücksichtigen, je geringer der Wirkstoffgehalt in der Tablette ist. Die erfindungsgemäßen Retardtablettenformulierungen weisen einen solchen geringen Wirkstoffgehalt auf. Zusätzlich ist es bekannt, dass Pramipexol durch seine elektrische Ladung während der Verarbeitung zu Agglomeration neigen kann, wodurch die Einheitlichkeit des Wirkstoffgehalts in den Tabletten ebenfalls sinkt.

Besonders bevorzugt weist die erfindungsgemäße Retardtablettenformulierung den Wirkstoff Pramipexol oder ein pharmazeutisch verträgliches Salz davon in einer Partikelgröße D₉₀ (mindestens 90 Volumenprozent der Partikel weisen einen Durchmesser auf, welcher geringer ist als der angegebene Wert) von weniger als 100 *µ*m, besonders bevorzugt weniger als 50 *µ*m, ganz besonders bevorzugt weniger als 20 *µ*m, am bevorzugsten weniger als 15 *µ*m auf.

Die Werte D₁₀, D₅₀ und D₉₀ der Partikel können auch verwendet werden, um die Partikelgrößenverteilung zu beschreiben. Entsprechend der oben getroffenen Definition besagt der Wert D₁₀, dass mindestens 10 Volumenprozent der Partikel einen kleineren Durchmesser als der angegebene Wert aufweisen, und der Wert D₅₀, dass mindestens 50 Volumenprozent der Partikel einen kleineren Durchmesser als der angegebene Wert aufweisen. Methoden zur Bestimmung dieser Partikelgrößen sind dem Fachmann bekannt. Die hier angegebenen Werte wurden mittels dynamischer Lichtstreuung mit einem Malvern Mastersizer 2000 gemessen.

Bevorzugt weist das Pramipexol oder pharmazeutisch verträgliche Salz davon eine Partikelgrößenverteilung von D₁₀ < 20 *µ*m, D₅₀ <50 *µ*m und D₉₀ < 100 *µ*m, besonders bevorzugt von D₁₀ < 10 *µ*m, D₅₀ <25 *µ*m und D₉₀ < 50 *µ*m und ganz besonders bevorzugt von D₁₀ < 5 *µ*m, D₅₀ <10 *µ*m und D₉₀ < 15 *µ*m auf. Mit dieser Partikelgrößenverteilung konnte eine einheitliche Wirkstoffverteilung In den resultierenden Matrixtabletten erzielt werden.

### (b) Matrix

Die erfindungsgemäße Retardtablettenformulierung umfasst eine Matrix umfassend 15 bis 35 Gew.-% Hydroxypropylmethylcellulose, 20 bis 48 Gew.-% Polyvinylacetat und 4,75 bis 11,40 Gew.-% Polyvinylpyrrolidon, jeweils bezogen auf das Gesamtgewicht der Retardtablettenformulierung. Besonders bevorzugt umfasst die Matrix 17 bis 33 Gew.-% Hydroxypropylmethylcellulose, 24 bis 44 Gew.-% Polyvinylacetat und 5,5 bis 10,5 Gew.-% Polyvinylpyrrolidon. Ganz besonders bevorzugt umfasst die Matrix 20 bis 30 Gew.-% Hydroxypropylmethylcellulose, 30 bis 42 Gew.-% Polyvinylacetat und 6 bis 10 Gew.-% Polyvinylpyrrolidon.

In einer bevorzugten Ausführungsform umfasst die Matrix 25 Gew.-% Hydroxypropylmethylcellulose, 36 Gew.-% Polyvinylacetat und 8,55 Gew.-% Polyvinylpyrrolidon.

Hydroxypropylmethylcellulose (HMPC) ist ein Stoffgemisch aus verschiedenen, teilweise substituierten Cellulosen. Es ist hydrophil und in Wasser sowie in den Verdauungssäften quellbar. Jedoch löst sich Hydroxypropylmethylcellulose auch mit der Zelt In den Verdauungssäften auf und erodiert somit. Der Matrixbildner HPMC fällt somit unter die oben beschriebene Gruppe (ii) der Gerüstbildner.

Bevorzugt weist die verwendete HPMC eine Viskosität von 100 bis 200 000 mPa*s, besonders bevorzugt von 1 000 bis 100 000, ganz besonders bevorzugt von 5 000 bis 30 000 mPa*s auf. Es ist auch möglich, dass Mischungen mehrerer HPMCs unterschiedlicher Viskosität verwendet werden können, wenn diese Mischung eine Viskosität von 100 bis 200 000 mPa*s, besonders bevorzugt von 1 000 bis 100 000, ganz besonders bevorzugt 5000 bis 30000 mPa*s aufweist. In einer bevorzugten Ausführungsform wird eine HPMC von 18 000 mPa*s verwendet. Methoden zur Bestimmung der Viskositäten von HPMC sind aus dem europälschen Arzneibuch bekannt.

Über die Viskosität der HPMC lässt sich die Freisetzungsrate der resultierenden Matrixtablette steuern. Dabei gilt: je höher die Viskosität (und damit auch die Molmasse) der HPMC, desto langsamer ist die Freisetzung. Gleichzeitig führen niedrigere Viskositäten der HPMC zu einer besseren Fließfähigkeit der Retardtablettenformulierung. Daher hat die Viskosität der HPMC einen entscheidenden Einfluss auf den Herstellungsprozess einer Matrixtablette sowie auf Ihr Freisetzungsprofil.

Polyvinylacetat (PVA) ist ein Material mit plastischen Verhalten, so dass bei einer Verpressung eine kohärente Matrix hoher Porosität selbst bei niedrigen Verpressungsdrücken erhalten wird. PVA Ist in den Verdauungssäften nicht löslich und gehört daher zu den oben beschriebenen Gerüstbildnern der Gruppe (iii).

Besonders bevorzugt wird das PVA erfindungsgemäß mit einer Molmasse von 100 000 bis 800 000 g/mol, ganz besonders bevorzugt 250 000 bis 650 000 g/mol, am bevorzugsten 400 000 bis 500 000 g/mol eingesetzt.

Das Matrixpolymer Polyvinylpyrrolidon (PVP) Ist in Wasser und den Verdauungssäften gut lösliche Substanz. Eine Matrixtablette aus PVP löst sich somit nach oraler Aufnahme im Magen auf und fällt daher unter die oben beschriebene Gruppe (i) der Gerüstbildner. In Kombination mit einem In den Verdauungssäften unlöslichen Gerüstbildner wie beispielsweise PVA, löst sich das PVP aus der Matrix heraus, wodurch in der unlöslichen Matrix zusätzliche Poren gebildet werden, durch die der Wirkstoff diffundieren und freigesetzt werden kann.

Besonders bevorzugt wird das PVP erfindungsgemäß mit einer Molmasse von 10 000 bis 90 000 g/mol, ganz besonders bevorzugt 25 000 bis 75 000 g/mol, am bevorzugsten 40 000 bis 60 000 g/mol eingesetzt.

Besonders bevorzugt umfasst die Retardtablettenformullerung der vorliegenden Erfindung PVA und PVP im einem Verhältnis von 6:4 bis 9:1, ganz besonders bevorzugt von 8:2 unter der Voraussetzung, dass die oben genannten Gew.-% der Gesamttablettenzusammensetzung realisiert werden.

Es hat sich überraschenderweise herausgestellt, dass durch die Verwendung der Kombination der drei Matrixpolymere HPMC, PVA und PVP in den angegebenen Mengenverhältnissen das Verfahren zur Herstellung einer Matrixtablette wesentlich vereinfacht werden kann. Diese Retardtablettenformulierung weist selbst bei variierender Wirkstoffkonzentration in einem Bereich von 0,05 bis 1 Gew.-% des Pramipexols berechnet als freie Base das gleiche Freisetzungsprofil auf. Somit können das Tablettengewicht und damit insbesondere auch die Tablettengröße konstant und daher unabhängig von der genannten Wirkstoffkonzentration sein. Dies führt dazu, dass bei der Herstellung der erfindungsgemäßen Matrixtabletten unabhängig von der Wirkstoffkonzentration jeweils die gleichen Verpressungsutensilien (Stempel, Matrizen etc.) sowie die gleichen Konfektionierungsutensilien (Blisterwerkzeuge etc.) benötigt werden. Dies reduziert die Totzeiten sowie die Kosten zur Bereitstellung unterschiedlicher Utensilien bei der Herstellung der Matrixtabletten. Des Weiteren steht dem Patienten bei der Einnahme immer die gleiche Größe an Tabletten zur Verfügung, wodurch Schwierigkeiten, die beim Herunterschlucken größerer Tabletten entstehen können, vermieden werden.

Ohne an eine Theorie gebunden sein zu wollen, wird davon ausgegangen, dass durch die Kombination der Matrixpolymere HPMC, PVA und PVP eine Kombination der drei Freisetzungsarten der Gruppen (i) bis (iii) erhalten werden kann. Es ist überraschend, dass durch die geeignete Kombination dieser Freisetzungsarten insbesondere die Diffusion des Wirkstoffs so beeinflusst werden kann, dass unabhängig von seiner Konzentration das gleiche relative Freisetzungsprofil erhalten werden kann. Normalerweise muss eine erhöhte Wirkstoffkonzentration einer Retardtablette mit einer Vergrößerung der Matrix, das heißt, einer Verlängerung der Diffusionswege, verbunden sein (siehe oben beschriebenes Sifrol^{®}), um dasselbe Freisetzungsprofil zu erhalten.

Unter dem gleichen Freisetzungsprofil wird im Sinne der vorliegenden Erfindung ein Frelsetzungsprofil verstanden, welches die gleichen Charakteristika, Insbesondere die gleiche Freisetzungsgeschwindigkeit (Steigung einer Auftragung der freigesetzten Menge des Wirkstoffes gegen die Zeit) aufweist. Wird in einer Auftragung die freigesetzte Menge des Wirkstoffes prozentual im Verhältnis zum In der Tablette enthaltenen Wirkstoffgehalt in Abhängigkeit von der Zeit aufgetragen, so lassen sich die relativen Freisetzungsprofile von Tabletten unterschiedlicher Wirkstoffkonzentration vergleichen.

Dabei ist es mit der erfindungsgemäßen Retardtablettenformullerung möglich, selbst bei unterschiedlichen Wirkstoffkonzentrationen in einer Tablette gleichen Gewichts gleiche relative Freisetzungsprofile des Wirkstoffs zu erhalten.

Bei dem Freisetzungsprofil handelt es sich um die in vitro Freisetzung. Das bedeutet, dass die Freisetzung gemeint Ist, wie sie erhalten wird, wenn eine für in vitro Experimente gängige Flüssigkeit verwendet wird, welche eine Freisetzung des Wirkstoffes bewirkt. Beispielsweise können hier Körperflüssigkeiten oder künstlich hergestellte Körperflüssigkeiten, insbesondere Verdauungsflüssigkeiten des Magen-Darm-Traktes verwendet werden. Solche Prüfflüssigkeiten werden im europäischen Arzneibuch beschrieben. Insbesondere können Prüfflüssigkeiten verwendet werden, welche ausgewählt werden aus der Gruppe, bestehend aus 0,1 N HCl (pH = 1,0), einem Phosphatpuffer (pH = 4,5; siehe europäisches Arzneibuch), einem Acetatpuffer (pH = 4,5; siehe europäisches Arzneibuch) und einem Phosphatpuffer (pH = 6,8; siehe europäisches Arzneibuch).

Des Weiteren hat sich herausgestellt, dass das Freisetzungsprofil der erfindungsgemäßen Retardtablettenformullerung pH-Wert unabhängig ist. Dies bedeutet, dass es im Wesentlich gleich ist, unabhängig vom pH-Wert. Dies hat insbesondere zur Folge, dass das Freisetzungsprofil unabhängig davon Ist, was der Patient zuvor gegessen oder getrunken hat.

Ebenso hat sich herausgestellt, dass die erfindungsgemäße Retardtablettenformulierungen bei unterschiedlichen Bruchfestigkeiten, beispielsweise durch Verwendung unterschiedlicher Pressdrücke, gleiche Freisetzungsprofile aufweisen. Auch damit können die bereits bekannten Verfahren des Standes der Technik vereinfacht werden. Insbesondere ist dadurch ein geringerer Pressdruck ausreichend, um das gleiche Freisetzungsprofil nach der Einnahme zu erzielen, wodurch Energie gespart wird. Des Weiteren können die Anforderungen an den Wassergehalt der zu verpressenden Bestandteile, welcher ebenso einen Einfluss auf die Bruchfestigkeit der Tablette hat, aufgeweitet werden. Dadurch ist ein solcher Herstellungsprozess stabiler und unempfindlicher. Des Weiteren ist bekannt, dass die Eigenschaften einiger Matrixpolymere auf Grund Ihrer niedrigen Glasübergangtemperaturen sich bei der Lagerung verändern und somit die Bruchfestigkeit und damit auch das Freisetzungsprofil negativ beeinflussen. Auch hier ist ein Vorteil der erfindungsgemäßen Retardtablettenformulierung zu sehen, da selbst bei sich verändernder Bruchfestigkeit der resultierenden Matrixtabletten bei der Lagerung dennoch im Wesentlichen das gleiche Freisetzungsprofil erhalten werden kann.

### (c) mindestens einen bei Tablettenformulierungen üblichen Bestandteil

Die erfindungsgemäße Retardtablettenformulierung umfasst (c) mindestens einen bei Tablettenformulierungen üblichen Bestandteil. Dieser ist dem Fachmann bekannt. Insbesondere umfasst die erfindungsgemäße Retardtablettenformulierung den mindestens einen bei Tablettenformullerungen üblichen Bestandteil zu so viel Gew.-%, dass sich zusammen mit dem Wirkstoff (a) und der Matrix (b) 100 Gew.-% der Retardtablettenformullerung ergeben.

Bevorzugt wird der mindestens eine bei Tablettenformulierungen übliche Bestandteil ausgewählt aus der Gruppe, bestehend aus Füllstoffen, Bindemitteln, Schmiermitteln, Fließregulierungsmitteln und Farbstoffen. Dieser mindestens eine bei der Tablettenformulierung übliche Bestandteil wird im Folgenden auch als "weiterer Bestandteil" oder "Hilfsstoff" bezeichnet.

Bevorzugt umfasst die erfindungsgemäße Retardtablettenformullerung mindestens 4,6 Gew.-%, besonders bevorzugt mindestens 11,5 Gew.-%, ganz besonders bevorzugt mindestens 17 Gew.-% mindestens eines weiteren Bestandteils, welcher ausgewählt wird aus der Gruppe, bestehend aus Füllstoffen, Bindemitteln, Schmiermitteln, Fließregulierungsmitteln und Farbstoffen, jeweils bezogen auf das Gesamtgewicht der Retardtablettenformulierung, Dies bedeutet, dass die erfindungsgemäße Retardtablettenformulierung bevorzugt bis zu 94,4 Gew.-% an Matrixpolymeren und bis zu 1 Gew.-% des Pramipexols berechnet als Base, besonders bevorzugt bis zu 87,5 Gew.-% an Matrixpolymeren und bis zu 1 Gew.-% des Pramipexols berechnet als Base und ganz besonders bevorzugt bis zu 82 Gew.-% an Matrixpolymeren und bis zu 1 Gew.-% des Pramipexols berechnet als Base umfasst und der verbleibende Anteil an Gewichtsprozenten bis 100 % mindestens einen weiteren Bestandteil, welcher ausgewählt wird aus der Gruppe, bestehend aus Füllstoffen, Bindemitteln, Schmiermitteln, Fließregulierungsmitteln und Farbstoffen, darstellt.

In einer bevorzugten Ausführungsform umfasst die erfindungsgemäße Retardtablettenformulierung 70 Gew.-% der genannten Matrix, 0,05 bis 1 Gew.-% des Pramipexols berechnet als Base und die verbleibenden Gewichtsprozente an mindestens einem weiteren Bestandteil, welcher ausgewählt wird aus der Gruppe, bestehend aus Füllstoffen, Bindemitteln, Schmiermitteln, Fließregulierungsmitteln und Farbstoffen.

Füllstoffe werden besonders bevorzugt ausgewählt aus der Gruppe, bestehend aus Stärken, Stärkehydrolysaten, Zuckern, Zuckeralkoholen, Cellulosen, Cellulosederivaten, anorganischen Calcium- oder Magnesiumverbindungen, synthetische Polymeren und beliebigen Mischungen davon. Dabei können unterschiedliche Zucker, wie beispielsweise Lactose Monohydrat, Lactose DT (für Direkttablettierung), wasserfreie Lactose, Flowlac™ (von Meggle Products), Pharmatose™ (von DMV) verwendet werden. In einer bevorzugten Ausführungsform enthält die Retardtablettenformulierung keine Lactose. Dies ist Insbesondere vorteilhaft, da auch Patienten mit Lactoseintoleranz die resultierenden Matrixtabletten einnehmen können. Unter Cellulosederivaten werden Insbesondere kristalline Cellulose und Cellulosepulver verstanden. Kommerziell erhältliche kristalline Cellulosen sind beispielsweise CEOLUS™ KG801, Avicel™ PH101, PH102, PH301, PH302, PH302, PH-112 mikrokristalline Cellulose 114, mikrokristalline Cellulose 112 und Prosolv™ (von JRS Pharma). In einer bevorzugten Ausführungsform umfasst die erfindungsgemäße Retardtablettenformulierung keine mikrokristalline Cellulose. Aus der WO 2011/037976 A2 ist bekannt, dass Pramipexol in pharmazeutischen Zusammensetzungen durch mikrokristalline Cellulose stabilisiert werden kann. Es wurde überraschenderweise gefunden, dass die erfindungsgemäße Retardtablettenformulierung sowie auch die resultierende Matrixtablette auch ohne die Anwesenheit von mikrokristalliner Cellulose stabil sind. Dies bedeutet, dass sich der Wirkstoff über einen Zeitraum von vorzugsweise 1 Jahr, besonders bevorzugt 2 Jahren, ganz besonders bevorzugt 3 Jahren nicht verändert. Weitere geeignete Füllstoffe können ausgewählt werden aus der Gruppe, bestehend aus Cellulosederivaten. Zuckeralkoholen wie Mannitol (Pearlitol™ SD200), Sorbitol und Xylitol, Calciumcarbonat, Magnesiumcarbonat, Calciumhydrogenphosphat als Dihydrat oder wasserfreie Variante und Calciumphosphat. Ganz besonders bevorzugt wird als Füllstoff Calciumhydrogenphosphat, wasserfrei verwendet. Dieses ist besonders geeignet zur Anwendung im Verfahren der Direktverpressung. Calciumhydrogenphosphat wird durch Sprödbruch verformt und ist weitgehend unempfindlich gegenüber Magnesiumstearat-Zusatz und Mischzeit. Insbesondere kann Calciumhydrogenphosphat eingesetzt werden, um eine lactosefreie Retardtablettenformulierung zu erhalten.

Besonders bevorzugt umfasst die erfindungsgemäße Retardtablettenformulierung als einen weiteren Bestandteil mindestens einen Füllstoff. Dabei umfasst die Retardtablettenformulierung bevorzugt mindestens 15 Gew.-%, besonders bevorzugt mindestens 20 Gew.-% eines Füllstoffes. In einer bevorzugten Ausführungsform ist der Füllstoff Calciumhydrogenphosphat. Ganz besonders bevorzugt umfasst die erfindungsgemäße Retardtablettenformulierung 27,0 bis 28,5 Gew.-% Calciumhydrogenphosphat.

Bindemittel werden besonders bevorzugt ausgewählt aus der Gruppe, bestehend aus Cellulosederivaten, Gelatine, Polysacchariden, , synthetischen Polymeren (z. B. Polymethacrylate, Polyacrylsäure, Povidone), Stärken, Stärkehydrolysaten und beliebigen Kombinationen davon. Selbstverständlich wirken die erfindungsgemäß eingesetzten Matrixpolymere HPMC, PVA und PVP insbesondere auch als Bindemittel. Daher umfasst die erfindungsgemäße Retardtablettenformulierung in einer bevorzugten Ausführungsform keine weiteren Bindemittel.

Schmiermittel werden bevorzugt ausgewählt aus der Gruppe, bestehend aus Salzen höherer Fettsäuren (z. B. Magnesiumstearat, Calciumstearat, Calciumbehenat), höheren Alkoholen (z. B. Stearylalkohol, Cetylstearylalkohol), Fettsäureestern (z. B. Glycerylmonostearat, Natriumlaurylsulfat) Fettsäuren (z. B. Palmitinsäure, Stearinsäure), Talkum, Carnaubawachs" Mono-, Di-, Triglyceriden höherer Fettsäuren (z. B. hydriertes Rizinussamenöl) und beliebigen Kombinationen davon. Bevorzugt umfasst die erfindungsgemäße Retardtablettenformulierung 0 bis 3 Gew.-%, besonders bevorzugt 0,5 bis 1,5 Gew.-%, ganz besonders bevorzugt 1 Gew.-% eines Schmiermittels. Ein bevorzugtes Schmiermittel ist dabei Magnesiumstearat.

Fließregulierungsmittel können bevorzugt ausgewählt werden aus der Gruppe, bestehen aus Siliciumdioxid, Talkum und beliebigen Mischungen davon. Die Fließregulierungsmittel werden eingesetzt, um die Fließfähigkeit des zu verpressenden Pulvers zu verbessern und dadurch die Einheitlichkeit der Zusammensetzung der resultierenden Matrixtablette zu gewährleisten. Bevorzugt umfasst die erfindungsgemäße Retardtablettenformulierung 0 bis 2 Gew.-%, besonders bevorzugt 0,1 bis 1 Gew.-%, ganz besonders bevorzugt 0,5 Gew.-% eines Fließregulierungsmittels. Besonders bevorzugt wird hochdisperses Siliciumdioxid als Fließregulierungsmittel eingesetzt.

Die erfindungsgemäße Retardtablettenformulierung kann auch einen Farbstoff umfassen, um die der resultierenden Matrixtablette eine Farbe zu verleihen, beispielswiese, um die Art und/oder Dosierung der Tablette zu kennzeichnen. Dabei werden bevorzugt natürliche und/oder künstliche Farbstoffe verwendet, welche ausgewählt werden aus der Gruppe, bestehend aus FD&C Farbstoffen, natürlichen Fruchtkonzentraten, Pigmenten wie beispielsweise Titandioxid, verschiedenen Eisenoxiden und beliebigen Mischungen davon.

In einer bevorzugten Ausführungsform besteht die erfindungsgemäße Retardtablettenformulierung neben der Matrix und Pramipexol oder einem pharmazeutisch verträglichen Salz davon aus einem Füllstoff, einem Schmiermittel und einem Fließregulierungsmittel.

Besonders bevorzugt besteht die erfindungsgemäße Retardtablettenformulierung neben der Matrix und Pramipexol oder einem pharmazeutisch verträglichen Salz davon aus Calciumhydrogenphosphat, wasserfrei, Magnesiumstearat und hochdispersem Siliciumdioxid.

In einer weiteren bevorzugten Ausführungsform besteht die erfindungsgemäße Retardtablettenformulierung aus
25,0 Gew.-% Hydroxypropylmethylcellulose,
36,0 Gew.-% Polyvinylacetat,
8,6 Gew.-% Polyvinylpyrrolidon,
28,0 Gew.-% Calciumhydrogenphosphat, wasserfrei,
0,5 Gew.-% hochdisperses Siliciumdioxid,
1,0 Gew.-% Magnesiumstearat,
0,07 bis 0,90 Gew.-% des Pramipexols berechnet als Base, wobei gegebenenfalls Spuren von Natriumlaurylsulfat enthalten sein können. Die variablen Gew.-% der Pramipexol Base können bevorzugt über die Variation des Gehalts an Calciumhydrogenphosphat, wasserfrei ausgeglichen werden. Dabei werden bevorzugt alle Gew.-% der anderen Inhaltsstoffe konstant gehalten.

Die erfindungsgemäße Retardtablettenformulierung kann als üblichen Hilfsstoff einen oder mehrere Stabilisatoren enthalten. Diese werden bevorzugt ausgewählt aus Ascorbinsäure und deren pharmazeutisch verträglichen Salzen und/oder Derivaten wie z.B. Fettsäureestern, z.B. Calciumascorbat, Natriumascorbat, Palmitoylascorbinsäure; Butylhydroxyanisol; Citronensäure; Natrlumedetat, Meglumin, Salzen von schwefliger Säure wie Natriummetabisulfit, Natriumsulfit, Kaliummetabisulfit; Tocopherol; Butylhydroxytoluol. Bevorzugte Stabilisatoren sind Ascorbinsäure, Butylhydroxyanisol, Citronensäure, Natriumedetat und Meglumin. Besonders bevorzugt sind auch Zweier- bzw. Dreierkombinationen von Stabilisatoren, insbesondere Ascorbinsäure/Butylhydroxyanisol, Ascorbinsäure/Citronensäure, Butylhydroxyanisol/Citronensäure oder Ascorbinsäure/Butylhydroxyanisol/Citronensäure.

Die Stabilisatoren werden der Retardtablettenformulierung ggf. bevorzugt in folgenden Mengen zugesetzt: Ascorbinsäure 0,01 bis 1,0 Gew.-%, bevorzugt 0,1 Gew.-%; Butylhydroxyanisol 0,005 bis 0,1 Gew.-%, bevorzugt 0,02 Gew.-%, Citronensäure 0,1 bis 3,0 Gew.-%, bevorzugt 1 Gew.-%, Natriumedetat 0,01 bis 0,5 %, bevorzugt 0,1 %, Meglumin 0,5 bis 5,0 %, bevorzugt 0,75 %. Im Falle von Stabilisatorkombinationen wird jede einzelne Komponente der Kombination bevorzugt in den zuvor angegebenen Mengen zugesetzt.

Es hat sich herausgestellt, dass die erfindungsgemäße Formulierung eine hohe Stabilität von bevorzugt mindestens 1 Jahr, besonders bevorzugt mindestens 2 Jahren, ganz besonders bevorzugt mindestens 3 Jahren aufweist.

### Matrixtablette

Ein weiterer Gegenstand der vorliegenden Erfindung Ist eine Matrixtablette, umfassend die erfindungsgemäße Retardtablettenformulierung.

Insbesondere ist die erfindungsgemäße Matrixtablette dadurch gekennzeichnet, dass sie durch Direktverpressung hergestellt wird. Darunter Ist insbesondere das Verpressen der pulverförmigen Retardtablettenformulierung ohne Vorbehandlung zu verstehen. Die Direktverpressung zeichnet sich insbesondere durch ein einfaches, mit geringem Arbeitsaufwand verbundenes Verfahren aus.

Bei der Direktverpressung wird bevorzugt mit einer Presskraft von 8 bis 15 kN, besonders bevorzugt von 9 bis 14 kN, ganz besonders bevorzugt von 11 bis 12 kN gearbeitet.

Die erfindungsgemäßen Matrixtabletten weisen bevorzugt eine Bruchfestigkeit von 120 bis 180 ± 10 N auf. Wie bereits oben ausgeführt, weisen die erfindungsgemäßen Matrixtabletten In diesem Bereich der Bruchfestigkeit im Wesentlichen das gleiche Freisetzungsprofil auf. Dabei kann die Bruchfestigkeit der Tabletten durch Methoden, wie sie beispielsweise im europälschen Arzneibuch (Ph. Eur. 2.9.8) beschrieben ist, bestimmt werden.

Bevorzugt weist die erfindungsgemäße Matrixtablette ein Gesamtgewicht von 240 mg bis 500 mg, besonders bevorzugt von 300 bis 400 mg und ganz besonders bevorzugt von 350 mg auf. Dabei ist das Gesamtgewicht der Tablette unabhängig von der in ihr enthaltenen Wirkstoffkonzentration im Bereich von 0,05 Gew.-% bis 1 Gew.-%, bezogen auf das Gesamtgewicht der Matrixtablette.

Die erfindungsgemäße Matrixtablette wird bevorzugt verwendet zur Behandlung der Parkinson-Erkrankung, des Restless-Legs-Syndroms und damit verbundene Störungen.

Ebenso ist Gegenstand der vorliegenden Erfindung ein Verfahren zur Herstellung der erfindungsgemäßen Matrixtablette, enthaltend Pramipexol oder ein pharmazeutisch verträgliches Salz davon, durch die folgenden Schritte:
(I) Vermischen von 0,05 Gew.-% bis 1 Gew.-% des Pramipexols berechnet als Base (a) mit mindestens einem Füllstoff und gegebenenfalls weiteren Hilfsstoffen;
(II) Vermischen von
   15 bis 35 Gew.-% Hydroxypropylmethylcellulose,
   20 bis 48 Gew.-% Polyvinylacetat und
   4,75 bis 11,40 Gew.-% Polyvinylpyrrolidon,
   jeweils bezogen auf das Gesamtgewicht der Retardtablettenformulierung, und gegebenenfalls weiterer Hilfsstoffe zur Bildung einer Matrix;
(III) Vermischen der Gemische aus den Schritten (I) und (II), gegebenenfalls unter Zugabe weiterer Hilfsstoffe;
(IV) Direkttablettlerung des Gemisches aus dem Schritt (III).

Die in Schritten (I), (II) und/oder (III) eingesetzten Hilfsstoffe entsprechen bevorzugt dem oben genannten mindestens einen bei Tablettenformulierung üblichen Bestandteil (c) (auch als weiterer Bestandteil bezeichnet). Dieser wird bevorzugt ausgewählt aus der Gruppe, bestehend aus Füllstoffen, Bindemitteln, Schmiermitteln, Fließregulierungsmitteln und Farbstoffen.

Besonders bevorzugt wird in Verfahrensschritt (I) ein Calciumhydrogenphosphat als Füllstoff eingesetzt. Bevorzugt wird in Verfahrensschritt (I) kein weiterer Hilfsstoff verwendet. In Verfahrensschritt (II) wird bevorzugt mindestens ein Fließregulierungsmittel als Hilfsstoff eingesetzt. Dabei ist das Fließregulierungsmittel besonders bevorzugt hochdisperses Sillciumdioxid. In Verfahrensschritt (III) wird bevorzugt mindestens ein Schmiermittel als Hilfsstoff hinzugegeben. Dieses Schmiermittel ist besonders bevorzugt Magnesiumstearat.

Zur Vermischung des Wirkstoffs und des mindestens einen Füllstoffs In Verfahrensschritt (I) sollten Methoden verwendet werden, welche eine homogene Verteilung des Wirkstoffes bewirken. Dies ist insbesondere notwendig, da 0,05 Gew.-% bis 1 Gew.-% des Pramipexols berechnet als Base eine geringe Menge an Wirkstoff in der Tablette darstellt und die Einheitlichkeit der Tablettendosierung gewährleistet sein muss. Insbesondere kann ein Zwangsmischer zur Vermischung verwendet werden.

Nach der Vermischung in Verfahrensschritt (II) kann gegebenenfalls ein weiterer Verfahrensschritt des Siebens der in Verfahrensschritt (II) erhaltenen Mischung durchgeführt werden, bevor die in Verfahrensschritt (II) erhaltene Mischung im Verfahrensschritt (III) eingesetzt wird.

Die Vermischung des Verfahrensschritts (III) wird bevorzugt in einem Freifallmischer durchgeführt.

Die Direkttablettierung des Verfahrensschritts (IV) wird bevorzugt mit einer Presskraft von 8 bis 15 kN, besonders bevorzugt von 9 bis 14 kN, ganz besonders bevorzugt von 11 bis 12 kN durchgeführt.

Nach dem Verfahrensschritt (IV) können sich optional weitere Verfahrensschritte der Konfektionierung anschließen. Diese sind dem Fachmann bekannt.

Das erfindungsgemäße Verfahren ist insbesondere gegenüber dem Stand der Technik von Vorteil, da zur Herstellung von Tabletten unterschiedlicher Wirkstoffkonzentration lediglich das Gemisch des Verfahrensschrittes (I) ausgetauscht werden muss, während alle anderen Verfahrensschritte inklusive der Maschinen und Maschinenutensilien beibehalten werden können. Dies führt Insbesondere zu einer Reduzierung der Totzeit bei Änderung der Wirkstoffkonzentration der herzustellenden Tablette.

### Beschrelbung der Bilder

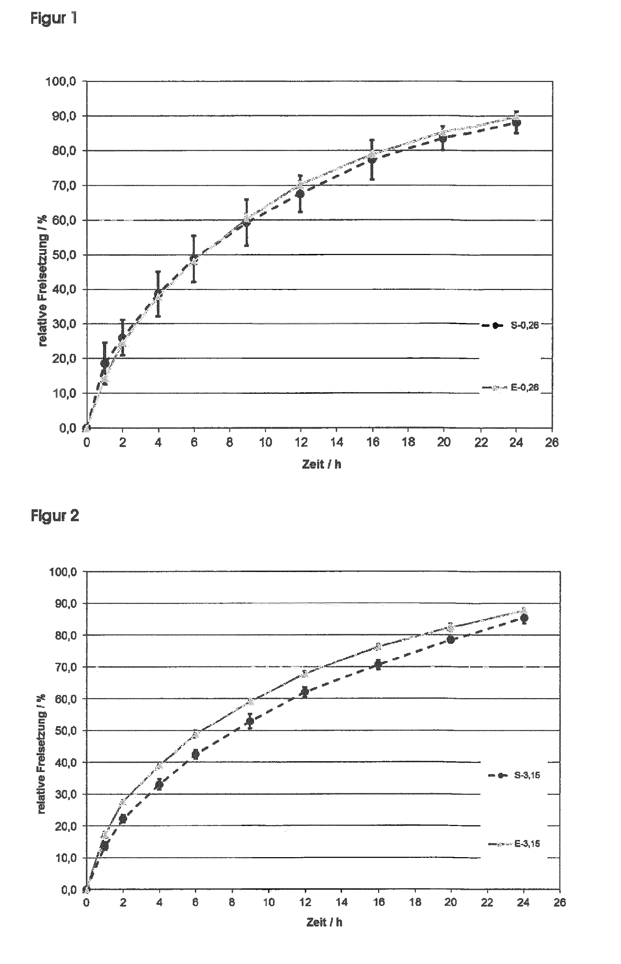

- Figur 1:: relatives Freisetzungsprofil (Messung siehe Beispiele) des Pramipexols in Abhängigkeit von der Zeit aus einer Tablette des Standes der Technik (S-0,26 enthaltend 0,26 mg Pramlpexol bei einem Tablettengewicht von 248 mg) und einer erfindungsgemäßen Tablette (E-0,26 enthaltend 0,26 mg Pramipexol bei einem Tablettengewicht von 350 mg).
- Figur 2:: relatives Freisetzungsprofil (Messung siehe Beispiele) des Pramipexols In Abhängigkeit von der Zeit aus einer Tablette des Standes der Technik (S-3,15 enthaltend 3,15 mg Pramipexol bei einem Tablettengewicht von 497 mg) und einer erfindungsgemäßen Tablette (E-3,15 enthaltend 3,15mg Pramipexol bei einem Tablettengewicht von 350 mg).

### Beispiele

### Herstellung einer erfindungsgemäßen Matrixtablette

Zur Bestimmung der Freisetzung des Wirkstoffs in einer Wirkstoffmenge von 0,26 mg und 3,15 mg Pramipexol Base (entspricht 0,07 Gew.-% oder 0,90 Gew.-% der Pramipexol Base beziehungsweise 0,375 mg oder 4,50 mg / 0,11 Gew.-% oder 1,29 Gew.-% Pramipexoldihydrochlorid Monohydrat) mit einem Tablettengewicht von jeweils 350 mg (im Folgenden als E-0,26 bzw. E-3,15 bezeichnet) wurde die folgende Matrixtablette hergestellt (soweit nicht anders angegeben beziehen sich die Gew.-% auf das Gesamtgewicht der Tablette; dabei werden die Gewichtsprozente des tatsächlich enthaltenen Pramipexoldihydrochlorid Monohydrates der Berechnung zu Grunde gelegt, so dass das Gesamtgewicht der Tablette 100 Gew.-% ergibt):

**Tabelle 2:**

| | **E-0,26** | **E-3,15** |
|---|---|---|
| | (erfindungsgemäße Tablette (350 mg) umfassend 0,26 mg der Pramipexol Base) | (erfindungsgemäße Tablette (350 mg) umfassend 3,15 mg der Pramipexol Base) |
| Pramipexoldihydrochlorid Monohydrat | 0,11 Gew.-% (entspricht 0,07 Gew.-% der Pramlpexol Base) | 1,29 Gew.-% (entspricht 0,90 Gew.-% der Pramipexol Base) |
| HPMC (Typ 2208, K15M PH CR) | 25 Gew.-% | 25 Gew.-% |
| Calciumhydrogenphosphat, wasserfrei | 28,39 Gew.-% | 27,21 Gew.-% |
| Kollidon SR* | 45 Gew.-% (entspricht 36 Gew.-% PVA und 8,55 Gew.-% PVP) | 45 Gew.-% (entspricht 36 Gew.-% PVA und 8,55 Gew.-% PVP) |
| Hochdisperses Siliciumdioxid | 0,50 Gew.-% | 0,50 Gew.-% |
| Magnesiumstearat | 1,00 Gew.-% | 1,00 Gew.-% |

| | | |
|---|---|---|
| * Marke der BASF AG: Mischung aus 80 % PVA, 19 % PVP, 0,8 % Natriumlaurylsulfat, 0,2 % SiO₂ | | |

Die angegebene Menge an Pramipexoldihydrochlorid Monohydrat wurde mit der jeweiligen Menge an Calciumhydrogenphosphat wasserfrei in einem Somacon-Zwangsmlscher (Rotor 350 UpM, Abstreifer: 20 UpM, Mischzeit 10 min) vermischt. Auf diesem Wege wurden fünf Vormischungen hergestellt, welche in einem Freifallmischer (Geschwindigkeit: 6 UpM, 5 min) vereinigt wurden. Dadurch wurde eine "Pramipexol Vormischung" erhalten.

Parallel dazu wurden die HPMC, das Kollidon SR sowie das hochdisperse Siliciumdioxid gesiebt (Durchmesser: 1,0 mm) und im Anschluss in einem Freifallmischer (6 UpM, 10 min) gemischt.

Die wie oben beschrieben erhaltene "Pramipexol Vormischung" wurde mit der Mischung aus HPMC, Kollidon SR und Siliciumdioxid in einem Frelfallmischer (Geschwindigkeit: 6 UpM, 20 min) vermischt. Im Anschluss wurde zu dieser Mischung in einem Freifallmischer Magnesiumstearat (zuvor gesiebt (Durchmesser: 0,7 mm)) gegeben und beides vermischt (Geschwindigkeit: 6 UpM, 5 min). Die so erhaltene Zusammensetzung stellt die pressfertige Mischung dar, welche im Anschluss zu 350 mg Tabletten (Durchmesser: 9 mm, gewölbt) mit 11 bis 12 kN verpresst wurde.

Als Vergleich wurde eine kommerziell erhältliche Tablette Sifrol^{®} 0,26 mg Retardtablette (Tablettengewicht: 248 mg) und Sifrol^{®} 3,15 mg Retardtablette (Tablettengewicht: 497 mg) (im Folgenden als S-0,26 bzw. S-3,15 bezeichnet) verwendet.

### Freisetzung des Pramipexols

Die Freisetzung der Tabletten E-0,26, E-3,15, S-0,26 und S-3,15 wurde in der Drehkörbchen-Apparatur (Apparatur 1) gemäß dem europäischen Arzneibuch bei 100 UpM in 500 mL eines Phosphatpuffers gemäß dem europäischen Arzneibuch bei einem pH-Wert von 6,8 über einen Zeitraum von 24 h mittels HPLC gemessen. Die Ergebnisse stellen Mittelwerte mit einer Standardabweichung von sechs Messungen dar und sind in der Figur 1 für eine Wirkstoffkonzentration von 0,26 mg Pramipexol und In Figur 2 für eine Wirkstoffkonzentration von 3,15 mg Pramlpexol dargestellt. Bei der Freisetzung handelt es sich um die relative Freisetzung, das heißt, die freigesetzte Menge an Pramipexol im Verhältnis zu der In der Tablette vorhandenen Gesamtmenge an Pramipexol.

Wie aus der Figur 1 ersichtlich ist, weisen die Tabletten E-0,26 und S-0,26 ein im Wesentlichen gleiches relatives Freisetzungsprofil des Pramlpexols auf. Dabei unterscheidet sich das Tablettengewicht von E-0,26 mit 350 mg zu dem von S-0,26 mit 248 mg deutlich. Ebenso weisen die Tabletten E-3,15 und S-3,15 bei einem unterschiedlichen Tablettengewicht von 350 mg zu 497 mg ein im Wesentlichen gleiches relatives Freisetzungsprofil auf. Weiterhin kann aus den Figuren geschlossen werden, dass die Tabletten E-0,26 und E-3,15 ein im Wesentlichen gleiches relatives Freisetzungsprofil aufweisen. Die erfindungsgemäße Matrix erlaubt es, dass das Tablettengewicht konstant gehalten werden kann und dennoch bei unterschiedlicher Wirkstoffkonzentration des Pramipexols das gleiche relative Freisetzungsprofil erhalten werden kann. Dies wird bei den Proben S-0,26 und S-3,15 nur durch die Veränderung des Gesamttablettengewichts möglich.

## Patentansprüche

1. Retardtablettenformulierung umfassend Pramipexol oder ein pharmazeutisch verträgliches Salz davon, **dadurch gekennzeichnet, dass** sie
(a) 0,05 Gew.-% bis 1 Gew.-% des Pramipexols berechnet als Base,
(b) eine Matrix und
(c) mindestens einen bei Tablettenformulierungen üblichen Bestandteil umfasst,
wobei die Matrix
15 bis 35 Gew.-% Hydroxypropylmethylcellulose,
20 bis 48 Gew.-% Polyvinylacetat und
4,75 bis 11,40 Gew.-% Polyvinylpyrrolidon,
jeweils bezogen auf das Gesamtgewicht der Retardtablettenformulierung, umfasst.

2. Retardtablettenformulierung nach Anspruch 1, wobei das pharmazeutisch verträgliche Salz des Pramipexols der BCS-Klasse I (Biopharmaceutics Classificatlon System) angehört.

3. Retardtablettenformulierung nach Anspruch 2, wobei der das pharmazeutisch verträgliche Salz des Pramipexols Pramipexoldihydrochlorid Monohydrat ist.

4. Retardtablettenformulierung nach einem der Ansprüche 1 bis 3, **dadurch gekennzeichnet, dass** der mindestens eine bei Tablettenformulierungen übliche Bestandteil (c) ausgewählt wird aus der Gruppe, bestehend aus Füllstoffen, Bindemitteln, Schmiermitteln, Fließregulierungsmitteln, Farbstoffen und Stabilisatoren.

5. Retardtablettenformulierung nach einem der Ansprüche 1 bis 4, **dadurch gekennzeichnet, dass** die Retardtablettenformulierung keine Lactose enthält.

6. Retardtablettenformulierung nach einem der Ansprüche 1 bis 5, **dadurch gekennzeichnet, dass** die Retardtablettenformulierung keine mikrokristalline Cellulose enthält.

7. Retardtablettenformulierung nach einem der Ansprüche 1 bis 6, **dadurch gekennzeichnet, dass** der/die Stabilisatoren ausgewählt wird aus der aus Ascorbinsäure und deren Salzen und Derivaten, Butylhydroxyanisol, Citronensäure, Natriumedetat, Meglumln, Salzen von schwefliger Säure, Tocopherol und Butylhydroxyanisol bestehenden Gruppe.

8. Matrixtablette, umfassend die Retardtablettenformulierung nach einem der Ansprüche 1 bis 7.

9. Matrixtablette nach Anspruch 8, **dadurch gekennzeichnet, dass** sie durch Direktverpressung hergestellt wird.

10. Verwendung der Matrixtablette nach einem der Ansprüche 8 oder 9 zur Behandlung der Parkinson-Erkrankung, des Restless-Legs-Syndroms und damit verbundener Störungen.

11. Verfahren zur Herstellung einer Matrixtablette, wobei das Verfahren die folgenden Schritte umfasst:
(I) Vermischen von 0,05 Gew.-% bis 1 Gew.-% des Pramipexols berechnet als Base (a) mit mindestens einem Füllstoff und gegebenenfalls weiteren Hilfsstoffen;
(II) Vermischen von
15 bis 35 Gew.-% Hydroxypropylmethylcellulose,
20 bis 48 Gew.-% Polyvinylacetat und
4,75 bis 11,40 Gew.-% Polyvinylpyrrolidon,
jeweils bezogen auf das Gesamtgewicht der Retardtablettenformulierung, und gegebenenfalls weiterer Hilfsstoffe zur Bildung einer Matrix;
(III) Vermischen der Gemische aus den Schritten (I) und (II), gegebenenfalls unter Zugabe weiterer Hilfsstoffe;
(IV) Direkttablettierung des Gemisches aus dem Schritt (III).
